# EUROPEAN PATENT SPECIFICATION

(11) **EP 0 735 895 B1**
(45) Date of publication and mention of the grant of the patent: **18.01.2006**
(21) Application number: 95900556.2
(22) Date of filing: 11.11.1994
(51) Int. Cl.: A61K 38/16, A61K 38/17, A61K 38/18, A61K 38/39, A61K 39/395, A61P 27/12

(54) **A METHOD FOR PREVENTING OR CONTROLLING CATARACT**
VERFAHREN ZUR PROPHYLAXE ODER KONTROLLE DES KATARAKTS
PROCEDE DE PREVENTION OU DE REDUCTION DE LA CATARACTE

(30) Priority: 19.11.1993 AU PM254093
(43) Date of publication of application: 09.10.1996
(73) Proprietor: THE UNIVERSITY OF SYDNEY, Sydney, New South Wales 2006 (AU)
(72) Inventor: McAVOY, Johnston William, Stanmore, NSW 2048 (AU); CHAMBERLAIN, Coral Gwenda, Five Dock, NSW 2046 (AU)
(74) Representative: Plougmann & Vingtoft A/S
(86) International application number: PCT/AU1994/000694
(87) International publication number: WO 1995/013827

(56) References cited:
- EP-A- 0 489 185
- WO-A-91/03990
- WO-A-91/04748
- WO-A-92/17206
- WO-A-93/09800
- WO-A-93/10808
- WO-A-93/19783
- WO-A-93/21945
- WO-A-94/01124
- WO-A-94/09815
- WO-A-94/10187
- AU-B2- 669 132
- GB-A- 2 250 442
- SU-A- 1 500 254
- PATENT ABSTRACTS OF JAPAN vol. 011, no. 080 (C-409), 11 March 1987 (1987-03-11) & JP 61 233622 A (RIKEN VITAMIN CO LTD), 17 October 1986 (1986-10-17)

## Description

### Technical Field

The present invention relates to the field of preventing or controlling pathological changes which occur in association with cataract formation in the mammalian eye by reducing the amount of or inhibiting the action of transforming growth factor-beta (TGFβ). The invention relates to the use of one or more inhibitors of TGFβ in the manufacture of a pharmaceutical formulation for preventing or controlling cataract or aftercataract formation.

### Background Art

Cataract is an opacity of the lens that interferes with vision. It is one of the most common of eye diseases and, though it may occur at any time in life, it often accompanies aging. In the USA, for example, up to 45% of people aged between 74 and 89 years suffer from cataract. Currently, the most commonly used treatment for cataract is surgical removal of the lens cells and subsequent implantation of a synthetic replacement lens within the remaining lens capsule. However, implantation of a synthetic lens may only temporarily restore vision because residual cells associated with the lens capsule often grow rapidly and form new opacities. The latter condition is known as "aftercataract" or post-operative capsular opacification.

The TGFβ family consists of a group of related proteins, the most extensively studied members being TGFβ1, TGFβ2 and TGFβ3 and it has been reported that these are all present in the eye.

### Disclosure of the Invention

In one aspect, the present invention provides the use of one or more inhibitors of TGFβ in the manufacture of a pharmaceutical formulation for preventing or controlling cataract of aftercataract formation.

Preferably, the mammalian subject is a human being but the present invention is also suitable for use in controlling cataract or after cataract formation in other animals such as horses, cats, dogs or the like.

Typically, the inhibitors of TGFβ are selected from proteins, glycoproteins and proteoglycans.

Suitable proteins include antibodies, peptide "growth factors" such as FGF, or the like.

Suitable glycoproteins include α₂-macroglobulin, laminin, collagen or the like.

Suitable proteoglycans include substances such as decorin, heparan sulfate proteoglycans, biglycan or the like.

In another aspect, the present invention provides a topical ophthalmological formulation for topical application to the surface of the eye for preventing or controlling cataract or aftercataract formation in the eye, said formulation comprising one or more inhibitors of TGFβ in an ophthalmologically acceptable carrier.

In yet another aspect, the present invention provides an ophthalmological irrigation solution or viscoelastic solution for preventing or controlling cataract or aftercataract formation in the eye comprising one or more inhibitors of TGFβ in an irrigation solution or viscoelastic solution.

In a further aspect, the present invention provides the use of one or more inhibitors of TGFβ in the manufacture of a synthetic lens for implantation into the lens capsule of the eye following the removal of lens cells from the lens capsule.

In yet another aspect, the present invention provides a synthetic lens implant for implantation into the lens capsule of the eye following removal of lens cells from the lens capsule, said lens implant being coated with one or more TGFβ inhibitors.

### Brief Description of Drawings

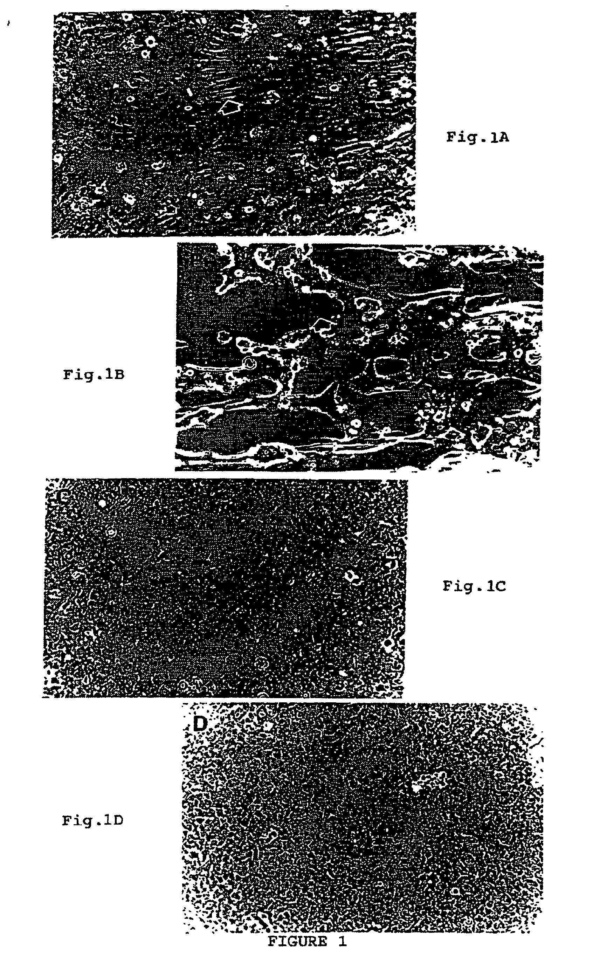

Figure 1 shows phase contrast micrographs of lens epithelial explants from 21-day-old rats cultured with TGFβ2 and non-immune IgG (A,B) or with TGFβ and anti-TGFβ IgG (C,D). Explants were photographed after 3 days (A,C) and 5 days (B,D) of culture.

### Modes for Carrving Out the Invention

The biological activity of TGFβ can be inhibited in a number of ways. One method of inhibiting the

biological activity is by using an antibody directed against an active region of the TGFβ molecule. TGFβ biological activity can also be inhibited by the use of other molecules which sequester, inhibit or inactivate TGFβ. For example, proteoglycans such as decorin act as specific TGFβ-binding proteins.

The present inventors have shown that the aqueous and vitreous that surround the lens of the eye contain molecules that inhibit the cataract-changes induced in lens cells by TGFβ and one or more of several inhibitory molecules mentioned above have been reported to be present in the occular media.

The TGFβ inhibitors can be administered either by topical application, by introduction into one or more chambers of the eye (for example, the anterior chamber), or as an intravenous injection at a site from which the inhibitors can be readily transported to the eye via the circulatory system. When the TGFβ inhibitor is α₂-macroglobulin, this can also be administered by mouth or by some other suitable route other than by way of an ophthalmological preparation. For example, it may be possible to provide lens cells with elevated levels of α₂-macroglobulin by administering a substance which causes an increase in serum levels of α₂-macroglobulin by affecting its synthesis or breakdown or enhancing its degree of transfer into the ocular media. Molecules related to α₂-macroglobulin ie, derived from it or specifically designed to mimic its TGFβ inhibitory properties but perhaps better able to pass through cellular membranes or the gut, may be administered in topical applications or by mouth or by other route.

The effective amount of the inhibitors of TGFβ required for use according to present invention will vary with the inhibitor used, with the route of administration, the stage of condition under treatment and the host undergoing treatment, and is ultimately at the discretion of the physician. Typically, the TGFβ inhibitors are presented as a pharmaceutical or ophthalmological formulation. The formulation can be used as.an adjunct to eye surgery to inhibit cataract-related changes that may occur as a result of surgical intervention as, for example, in the formation of ."aftercataract" following implantation of synthetic lens material. The present invention may also be suitable for individuals otherwise at greater than normal risk of cataract formation or of being exposed to elevated TGFβ levels near the lens.

Most of the inhibitors of TGFβ mentioned above are commercially available.

Decorin and biglycan can be obtained by purification according to Choi et al. Note that PGI and PGII are synonyms for biglycan and decorin respectively.

Heparan sulfate proteoglycans can be obtained according to the method of Yanagishita et al.

Ophthalmological formulations of the present invention are prepared according to conventional pharmaceutical formulating techniques. The carrier may be of any form depending on the form of preparation desired for administration and the formulation may optionally contain other therapeutic ingredients. Typically, one or more inhibitors of TGFβ can be included in conventional irrigation solutions or viscoelastic solutions. Lens implants coated with one or more TGFβ inhibitors may contain other therapeutic agents and may be prepared according to conventional techniques.

### EXAMPLE 1.

### Influence of TGFβ alone and in combination with FGF on lens epithelial explants.

### METHODS

Lens explants were prepared from both postnatal and adult rats and changes during 5 days culture with growth factor(s) were monitored by light and electron microscopy, immunolocalisation of laminin, heparan sulphate proteoglycan and fibre-specific crystallins, and crystallin ELISAs.

Each experiment involved culturing explants for up to 5 days without added growth factors (controls), with TGFβ, with a combination of TGFβ and FGF (TGFβ/FGF), or with FGF alone. FGF is another growth factor that influences lens cell behaviour (Chamberlain and McAvoy, 1989; McAvoy et al., 1991). In some experiments, explants were prepared by a standard method used in our laboratory in which the adhering capsule serves as the substratum for the cells. In others, explants were inverted onto a laminin substratum. The latter method allows cell attachment, spreading and migration to be monitored as well as providing good visualisation of individual cells.

Bovine brain basic FGF was prepared and stored at -20°C as described by Chamberlain and McAvoy (1989). Ultrapure natural human TGFβ1 was obtained from Genzyme (Cambridge, MA) and stored at -80°C. Working stock solutions of TGFβ and FGF were prepared (in culture medium or 1% bovine serum albumin-0.5 M NaCl in phosphate-buffered saline, respectively) and centrifuged at 10,000 g for 10 min at 4°C just before use.

### Preparation and Culture of Lens Epithelial Explants: Standard Method

Eyes were removed from 10-day-old and 14-week-old Wistar rats under sterile conditions and placed in medium, that is, medium 199 containing bovine serum albumin and antibiotics as described by Hales et al (1992), pre-incubated at 37°C in 5% CO₂/air. Lenses were removed and incubated in 2 ml medium for 45-90 min (postnatal) or 1-2 hr (adult). Epithelia were then peeled away from fibres and pinned out with the cellular surface uppermost in culture dishes containing 2 ml medium as described by McAvoy and Fernon (1984). The whole epithelium was used, unless otherwise specified, and each dish contained 2-3 explants.

Approximately 3 hr after preparation of explants, medium was replaced (1 ml/dish) and 10 µl samples of stock solutions of TGFβ and/or FGF were added, as required, to give final concentrations of 20 and 40 ng/ml, respectively. Explants were cultured for 5 days with daily monitoring by phase contrast microscopy. At appropriate times explants were processed for light or electron microcopy as described below. Alternatively, to assess the accumulation of fibre-specific crystallins, at the end of the culture period, explants were placed in 10 mM EDTA-0.02% Triton X-100, pH 10 (two explants in 200 µl) and stored at -20°C, then used for β- and γ-crystallin ELISAs with standards ranging from 0-20 ng/well.

### Preparation and Culture of Lens Epithelial Explants: on Laminin Substratum

This method is as described by Hales et al (1992). Briefly, on the day before the experiment, culture dishes were pre-coated with laminin. Whole explants were then prepared as described above, but with the cellular surface placed face down on the laminin and using lenses from 21-day-old rats; explants from rats of this age show a strong migratory response to FGF (unpublished observation). Each dish contained three explants. Growth factor treatments and culture conditions were as described for standard explants, except that a lower concentration of FGF, 2 ng/ml, was used to ensure that the main response to FGF alone was cellular migration rather than fibre differentiation. Responses were monitored daily by phase contrast microscopy.

### Microscopy

Explants used for immunofluorescent localisation were collected at the end of the culture period, fixed in Carnoy's fixative for 20 min at room temperature, transferred to 70% ethanol, then covered with a drop of melted 2.5% agar, before dehydrating in ethanol and embedding in paraffin. Sections were cut perpendicular to the explant surface and stained with haematoxylin-phloxine or used for immunolocalisation of laminin, heparan sulphate proteoglycan (HSPG) or β- and γ-crystallins. For each antibody and each explant 20-30 sections cut through the central region were examined, and at least two explants were processed for each growth factor treatment. Controls for non-specific fluorescence were included routinely, that is, sections were treated with non-immune rabbit serum instead of specific antibody. For whole mounts, explants were fixed in the culture dish with 100% ethanol and stained with haematoxylin-phloxine.

For ultrastructural studies, explants from 10-day-old rats were processed for transmission electron microscopy (TEM) and for scanning electron microscopy (SEM) as described by Lovicu and McAvoy (1992); explants were collected at 3 or 5 days of culture. Explants from adult rats were processed for SEM only at 5 days. For both SEM and TEM, at least two explants were viewed for each treatment and, for TEM, 20-30 grids were viewed per explant.

### RESULTS

Epithelial explants from postnatal rats (10 and 21 days old) were used for initial detailed studies. Because of the unusual nature of the observed responses to TGFβ, a brief comparative study was then carried out using explants from adult rats.

### Lens Explants from 10-day-old Rats: Standard Method

**Phase contrast microscopy and SEM.** In control and TGFβ-treated explants the cells retained a characteristic epithelial cell morphology throughout the culture period, that is, they were present in a monolayer with cobblestone-like packing. In both cases, some cell debris was detected on the monolayer surface. In TGFβ-treated explants only, single cells or small groups of cells were also occasionally detected on the monolayer surface. SEM of explants cultured for 5 days showed that the apical surface of some cells in TGFβ-treated explants overlapped onto neighbouring cells.

TGFβ/FGF- and FGF-treated explants were clearly distinguishable from controls within the first day of culture and indistinguishable from each other at this stage. Cells were irregularly packed and intercellular spaces were common, an explant morphology that is generally associated with active cell migration (McAvoy and Chamberlain, 1989; McAvoy, 1988). After 2 days culture some cells in the TGFβ/FGF-treated, but not in the FGF-treated, explants were extensively elongated. The number of elongated cells varied between explants; they generally formed only a small proportion of the cellular population but because they often formed regular rows they were quite distinct from the other cells in the explant which appeared similar to those in the FGF-treated explants. This marked difference between treatments was even more apparent at 3 days culture due to more cells becoming extensively elongated in TGFβ/FGF-treated explants. At this stage SEM showed that many of the elongated cells were attached to neighbouring cells at multiple sites along their length.

By 4 and 5 days culture, most of the cells in TGFβ/FGF-treated explants were in multilayers and all these explants had developed several regions where the cells were arranged in rosettes with elongated cells radiating out in a circular array from a focal point. Outside these rosettes, which occupied up to about 50% of the explant surface, there were some areas where similar extensively elongated cells were arranged in parallel arrays. Remaining cells were less elongated and appeared irregularly arrayed as in FGF-treated explants.

SEM showed that, in regions outside the rosettes and parallel arrays of extensively elongated cells, cells had numerous interlocking processes and appeared similar to the early differentiating fibres seen in explants treated with FGF alone. The morphological changes in explants from 10-day-old rats undergoing fibre differentiation in response to this concentration of FGF have been reported in detail elsewhere (Lovicu and McAvoy, 1992); multilayering and the formation of numerous interlocking processes are well-established features of this process (Lovicu and McAvoy, 1992; Lovicu and McAvoy, 1989). In the FGF/TGFS-treated explants, occasional patches of fibrillar extracellular matrix (ECM)-like material were noted on the explant surface. This matrix was dense and obscured the cells below.
**TEM.** Cells in explants cultured with FGF and TGFβ/FGF for 5 days became multilayered and exhibited features of early fibre differentiation including elongation, sparse cytoplasmic organelles and nucleolar RNA particle aggregations; ball-and-socket joints typical of fibre differentiation were also detected. Additionally in TGFβ/FGF-treated explants, cells exhibiting margination of chromatin and cytoplasmic condensation were common, and membrane-bound cellular fragments and electron-dense bodies resembling secondary lysosomes were found within many cells that otherwise appeared normal. These features are characteristic of apoptosis or programmed cell death (Wyllie et al. 1980; Williams et al., 1992). Similar apoptotic changes were also detected in TGFβ/FGF-treated explants at 3 days.

Pockets of ECM-like granular material were commonly detected between cells (and sometimes appeared to be within cells) in TGFβ/FGF-treated explants. Often near the cell membrane this material was present in a laminar arrangement and coated pits and vesicles were common in such regions. Cells with prominent rough endoplasmic reticulum and Golgi, which also usually showed abundant arrays of microfilaments, were also found frequently in these explants.

In explants cultured with TGFβ alone, the epithelial cells remained in a monolayer and were similar to controls except that, in the presence of TGFβ, spaces were often present between cells. This, together with the overlapping of cells suggests that TGFβ may be causing some disturbance of cell-cell interactions. **Immunohistochemical localisation of laminin and HSPG.** The ECM molecules laminin and HSPG are both found in the normal lens capsule (Parmigiani and McAvoy, 1991; Mohan and Spiro, 1986) and, as expected, reactivity for both laminin and HSPG was detected in the capsule in all explants irrespective of treatment.

In TGFβ/FGF-treated explants, reactivity for both laminin and HSPG was also localised within the explant in sites that were approximately similar in size and distribution to the pockets of ECM-like material seen by TEM. In FGF-treated explants, a few such regions were also detected; however, these were generally smaller and not as numerous as in explants treated with both growth factors. More sites exhibited reactivity for laminin than for HSPG and generally laminin reactivity was stronger.

In controls and TGFβ-treated explants no pockets of reactivity for laminin or HSPG were detected within the cellular layer. Thus the intercellular spaces revealed by TEM in TGFβ-treated explants did not contain ECM. **β-crystallin accumulation.** To assess fibre differentiation we measured the fibre-specific β- and γ-crystallin content of explants at the end of the 5 day culture period by ELISA. Significant β-crystallin accumulation occurred only in explants cultured with TGFβ/FGF or FGF (P = 0.001, compared with control); an apparent enhancement of β-crystallin accumulation in TGFβ/FGF-treated explants relative to the FGF-treated explants did not reach statistical significance. None of the treatments induced significant accumulation of γ-crystallin within the 5 day culture period.

Complementary immunolocalisation studies confirmed these findings and revealed that β-crystallin appeared to be distributed throughout most cells in both TGFβ/FGF-and FGF-treated explants.

### Lens Explants from 21-day-old Rats: on Laminin Substratum

When explants were cultured cell surface down on a laminin substratum without growth factors, cells spread and migrated off the capsule onto the substratum forming an annulus around the explant. This process continued over the 5 day culture period and was significantly enhanced by FGF (Hales et al., 1992). The addition of TGFβ, however, inhibited spreading and migration in the presence or absence of FGF so that a full annulus of cells did not develop; there were only a few isolated outgrowths of cells around the explant perimeter, and spreading and migration appeared to cease after 2 days of culture. This is consistent with the observation that the cells at the leading edge of these outgrowths had few of the pseudopodia characteristic of rapidly migrating cells seen in FGF-treated explants at 2 days. There was no apparent difference between TGFβ- and TGFβ/FGF-treated explants throughout the culture period.

During the first day of culture, all the cells in TGFβ-treated explants (that is, with or without FGF) had a morphology very similar to those in controls; however, by day 2 most of the cells that had spread onto the laminin substratum had become substantially elongated, some to the extent of being spindle-shaped or needle-like. In some regions cells that remained under the capsule also become elongated and aligned; these regions tended to extend between islands of epithelial-like cells. By 3 days of culture, explants treated with TGFβ mostly consisted of elongated cells and under the capsule differences between the peripheral and central regions of the explants became detectable. The periphery was well populated with multilayers of aligned elongated cells, whereas cells in the central region were in reticular arrangements exposing regions of bare capsule.

Wrinkling of the capsule was noted in all explants cultured with TGFβ under these explant conditions. The wrinkles had a reticular arrangement and were primarily located in the central region of the explant. The wrinkles were most obvious at 2 days and generally became less pronounced during the remainder of the 5 day culture period.

Cell loss also appeared to be a major feature of explants exposed to TGFβ**.** Bare patches of capsule were initially detected in the central region of the explant at 3 days and condensed nuclei were readily visible in cells that had spread onto the laminin. Cell numbers then progressively decreased and by 5 days the majority of the cells had been lost from the explant; the remaining cells retained the reticular arrangement first observed at 3 days.J

### Lens Explants from Adult Rats: Standard Method

**Phase contrast and SEM.** The morphological changes observed by phase contrast microscopy in these experiments were essentially similar to those reported for the explants from 21-day-old rats cultured on laminin, although as expected under these culture conditions no cells migrated off the capsule. Throughout the culture period there were no clear differences between TGFβ- and TGFβ/FGF-treated explants. During day 1, explants cultured with TGFβ retained the cobblestone appearance characteristic of controls, but by 2 days many of the cells had elongated. Bare patches of capsule were detected at 3 days and these increased progressively during the culture period.

The latter finding was confirmed by SEM at 5 days which also revealed that the morphology of cells that remained in explants cultured with TGFβ for 5 days was variable. Often cells were present in reticular arrays which seemed to consist mainly of mosaics of cells many of them epithelial-like. In other regions many cells were elongated and distinctly spindle- or needle-like and in some of these the cellular surface was covered with fine blebs. In the explant periphery, where more cells tended to survive, they were often present either as multilayers of smooth surfaced spindle-shaped cells or as more rounded cells with distinct surface blebbing typical of cells undergoing apoptotic cell death (Wyllie et al., 1980; Williams et al., 1992).

In FGF-treated explants, most cells retained an epithelial morphology although in the periphery some cells showed slight elongation characteristic of early fibre differentiation (Lovicu and McAvoy, 1992). Controls stayed as an epithelial monolayer throughout the culture period.
**Immunohistochemical localisation studies.** The pockets of laminin or HSPG reactivity reported above were not detected in explants from adult rats examined at the end of the culture period, irrespective of treatment. Reactivity for β-crystallin was detected in some cells interspersed throughout the explant in controls and FGF-treated explants; in both TGFβ and TGFβ/FGF-treated explants the clumps of cells that survived for 5 days also included some cells that fluoresced for β-crystallin. No γ-crystallin was detected in any of the explants. There was thus no evidence that any of the treatments stimulated ECM production or fibre-specific crystallin accumulation during the 5 day culture period.

### SUMMARY

TGFβ induced cells in explants to undergo an extensive and rapid elongation which had features that distinguished it from FGF-induced fibre differentiation. TGFβ also induced accumulation of extracellular matrix, capsule wrinkling, cell death by apoptosis and distinctive arrangements of cells. These TGFβ-induced responses are characteristic of the changes reported to occur during formation of various types of cataracts (Novotny and Pau, 1984; Eshagian, 1982; Eshagian and Streeten, 1980; Green and McDonnell, 1985). Standard explants from 10-day-old rats responded to TGFβ only in the presence of FGF. Comparable explants from adult rats, or from 21-day-old rats cultured on a laminin substratum, responded readily to TGFβ whether or not FGF was present.

### EXAMPLE 2

### Detailed description of an explant study using an antibody against TGFβ to inhibit TGFβ-induced cataract-like changes.

### METHOD

Lens epithelial explants (2 per culture dish) were prepared from 21-day-old rats and trimmed to remove the peripheral region as described elsewhere (See Example 1, Standard Method). Explants were preincubated in culture medium at 37°C in 5% CO₂/air for approximately 3 hours before use.

A pan-specific polyclonal antibody against TGFβ (rabbit IgG; British Bio-technology, Abingdon, UK; Cat. No. BDA 47,) was used; this neutralises TGFβ1, β1.2, β2, β3, and β5. This IgG and non-immune rabbit IgG were reconstituted in sterile phosphate-buffered saline to a concentration of 3 mg IgG/ml.

TGFβ2 (Genzyme, Cambridge, MA) was diluted with sterile medium to a concentration of 0.25 ng/10 µl. Under sterile conditions, 33 µl immune or non-immune IgG solution was mixed with 20 µl TGFβ2 stock solution and 47 µl medium, incubated at 37°C in 5% CO₂/air for 30 min, then diluted to 2 ml with medium. Preincubation medium was removed from two culture dishes and 1 ml TGFβ-IgG mixture was added to each. All explants were cultured for 5 days with daily monitoring by phase contrast microscopy. Explants cultured with non-immune IgG served as controls for any effects of IgG itself on TGFβ activity.

Figure 1 shows phase contrast micrographs of lens epithelial explants from 21-day-old rats cultured with TGFβ2 and non-immune IgG (A,B) or with TGFβ and anti-TGFβ IgG (C,D). Explants were photographed after 3 days (A,C) and 5 days (B,D) of culture. TGFβ induces extensive elongation of cells (A, arrow); subsequently many cells are lost exposing regions of capsule which show wrinkles (B, arrow). Anti-TGFβ completely blocks these changes and epithelial cells remain in a normal closely packed cobble-stone arrangement (C,D). The final concentrations of TGFβ and IgG were 0.25 ng/ml and 50 µg/ml, respectively.

### RESULTS

In the presence of non-immune IgG, TGFβ induced rapid elongation which occurred within 2-3 days (Fig. 1A) and by 5 days cells had been lost from the explant revealing wrinkling of the underlying capsule (Fig. 1B). These changes are typical of changes described in detail in Example 1 for explants cultured with TGFβ in the absence of IgG.

In the presence of anti-TGFβ, these changes were completely blocked. Throughout the 5 day culture period the explants retained their original epithelial-like morphology (Fig. 1C, D) and were indistinguishable from explants cultured in medium alone.

### EXAMPLE 3

### Detailed description of an explant study using aqueous and vitreous to inhibit TGFβ-induced cataract-like changes

### METHOD

Aqueous and vitreous were obtained from the eyes of freshly slaughtered 2 to 3 year-old-cattle as follows. Immediately on removal of the eye, the aqueous (about 1.5 ml) was collected using a sterile syringe fitted with a 23 gauge needle and an incision was made around the cornea to gain access to the lens. After carefully removing adhering iris, the lens was lifted out and the vitreous adjacent to the lens, mainly liquid vitreous, was collected (2-3 ml) using a syringe without needle and taking care to avoid contamination with retina. The whole procedure was completed within about 1 hour of the death of the animals. Samples were transported to the laboratory on ice and used as soon as possible.

Lens epithelial explants (2 per culture dish) were prepared from 21-day-old rats as described previously (See Example 1, Standard Method). Samples of aqueous or vitreous were diluted with an equal volume of sterile culture medium (defined in Example 1), using repeated passage through a 23 gauge needle to ensure thorough mixing. These mixtures were equilibrated at 37°C for 30 minutes in 5% CO₂/air before use. Stock solutions containing 25 or 100 pg/10µl TGFβ2 (Genzyme, Cambridge, MA) were prepared in sterile medium. Nine treatment groups were then set up by replacing medium in culture dishes containing explants with 1 ml medium or diluted aqueous or vitreous, with or without added TGFβ, as indicated in Table 1. Explants were cultured and monitored for cataract-like changes by phase contrast microscopy. In particular, each explant was graded according to the extent of spindle-like elongation and cell death, which are characteristically observed in explants cultured with TGFβ (See Examples 1 and 2). Explants were photographed on day 4.

### RESULTS

No significant changes were observed for any treatment on day 1; explants retained typical epithelial morphology. Explants cultured with medium alone did not change throughout the culture period. With continuing culture, explants cultured with TGFβ showed typical cataract-like changes, with more cell death for 100 pg/ml than 25 pg/ml (Table 1). Aqueous virtually completely inhibited the effects of 25 pg/ml TGFβ and partially inhibited the effects of 100 pg/ml. (With or without TGFβ, aqueous also caused some shrivelling of the capsule.) Explants cultured with vitreous, with or without TGFβ, showed changes typical of early fibre differentiation (cf. Schulz et al., 1993), but there was no evidence of cataract-like changes; vitreous thus completely inhibited the cataract-like changes induced by TGFβ.

**Table 1. Inhibition of TGFβ-induced cataract-like changes in rat lens epithelial explants by aqueous and vitreous**

| Treatment | TGFβ2 concentration (pg/ml) | | |
|---|---|---|---|
| | 0 | 25 | 100 |
| *Day 3*: | | | |
| Culture medium | - | +++/†† | ++++/†† |
| Aqueous | - | - | ++/†† |
| Vitreous | fd | fd | fd |

| *Day 4:* | | | |
|---|---|---|---|
| Culture medium | - | o/tttt | o/†††† |
| Aqueous | - | - | o/†††† |
| Vitreous | fd | fd | fd |

| *Day 5:* | | | |
|---|---|---|---|
| Culture medium | - | o/tttt | o/†††† |
| Aqueous | - | - | o/†††† |
| Vitreous | fd | fd | fd |

| | | | |
|---|---|---|---|
| Explants were cultured with medium or with diluted aqueous or vitreous, with or without the addition of TGFβ, as indicated. Four explants were subjected to each treatment. Code: -, negligible change; + - ++++, indicates extent of spindle-like elongation; † - ††††, indicates extent of cell loss; o, elongation assessment was invalidated by cell loss; fd, changes typical of early fibre differentiation with no cataract-like changes. | | | |

### SIGNIFICANCE

This study indicates that the aqueous and vitreous that surround the lens of the eye contain molecules that inhibit the cataract-like changes induced in lens cells by TGFβ. This effect may be due to the presence of one or more of several different kinds of inhibitory molecules which have been reported to be present in aqueous and vitreous: e.g. serum proteins, such as α₂₋macroglobulin; proteoglycans, such as decorin or heparan sulphate proteoglycans; or other peptide 'growth factors' such as FGF. All these molecules have been reported to bind to and/or inhibit TGFβ activity (LaMarre et al., 1991; Yamaguchi et al., 1992; McCaffrey et al., 1992; Hales et al, in press). α₂-macroglobulin is synthesised by the cornea (Twining et al. 1994) and is present in the aqueous (Ando et al., 1993); it probably enters the aqueous and vitreous along with other serum proteins found in these media (see, for example, Beebe et al., 1986). It has been shown that decorin is present near the lens in proliferative vitreoretinopathy (Hagedorn et al., 1993). Heparan sulphate is present in the vitreous, probably in association with extracellular matrix proteoglycans (Kamei et al., 1992). FGF is reported to be present in vitreous, and in much lower amounts in aqueous (Schulz et al., 1993), and to suppress the formation of TGFβ-induced spindle-cell formation in lens explants (Hales et al, in press). Other molecules known to bind to TGFβ and/or inhibit its activity, which may be contributing to the observed inhibitory effects of aqueous and/or vitreous, include biglycan (Yamuguchi et al., 1990), laminin and collagen (Paralkar et al., 1991).

### EXAMPLE 4

### Detailed description of an explant study using α₂₋macroglobulin to inhibit TGFβ-induced cataract-like changes

### METHOD

α₂-macroglobulin prepared from bovine plasma was obtained from Boehringer Mannheim Australia (Castle Hill, NSW; #602 442). Lens epithelial explants (2 per culture dish) were prepared from 21-day-old rats as described previously (See Example 1, Standard Method). α₂₋macroglobulin was dissolved in culture medium (defined in Example 1) at a final concentration of 400 µg/ml. The solution was sterilised by passing through a 0.22 µm filter and a portion was diluted with an equal volume of sterile medium. These solutions were equilibrated at 37°C in 5% CO₂/air before use. A stock solution containing 25 pg/10µl TGFβ2 (Genzyme, Cambridge, MA) was prepared in sterile medium. Six treatment groups were then set up by replacing medium in culture dishes containing explants with 1 ml medium or 1 ml medium containing 200 or 400 µg/ml α₂-macroglobulin, with or without added TGFβ, as indicated in Table 2. Explants were cultured and monitored for cataract-like changes by phase contrast microscopy. In particular, each explant was graded according to the extent of spindle-like elongation and cell death, which are characteristically observed in explants cultured with TGFβ (See Examples 1 and 2). Explants were photographed on days 3-5.

**Table 2. Inhibition of TGFβ-induced cataract-like changes in rat lens epithelial explants by α₂₋macroglobulin**

| Treatment | TGFβ2 concentration (pg/ml) | |
|---|---|---|
| | 0 | 25 |
| *Day* 2: | | |
| Culture medium | - | + |
| α₂-MG, 200 µg/ml | - | - |
| α₂-MG, 400 µg/ml | - | - |

| *Day 3:* | | |
|---|---|---|
| Culture medium | - | +++/† |
| α₂ -MG, 200 µg/ml | - | + |
| α₂-MG, 400 µg/ml | - | + |

| *Day 4:* | | |
|---|---|---|
| Culture medium | - | o/†††† |
| α₂-MG, 200 µg/ml | - | +/† |
| α₂-MG, 400 µg/ml | - | +/† |

| *Day 5:* | | |
|---|---|---|
| Culture medium | - | o/†††† |
| α₂-MG, 200 µg/ml | - | -/† |
| α₂-MG, 400 µg/ml | - | -/† |

| | | |
|---|---|---|
| Explants were cultured with medium, with or without the addition of α₂-macroglobulin (α₂-MG) and/or TGFβ, as indicated. Four explants were subjected to each treatment. Code: -, negligible change or reverted to predominantly epithelial morphology; + - ++++, indicates extent of spindle-like elongation; † - ††††, indicates extent of cell loss; o, elongation assessment was invalidated by cell loss. | | |

### RESULTS

### No significant changes were observed in explants cultured with medium alone or with medium containing α₂₋macroglobulin; the explants retained typical epithelial morphology throughout the culture period. On days 2-5, explants cultured with TGFβ showed typical cataract-like changes (Table 2). The TGFβ-induced changes were substantially inhibited by including α₂-macroglobulin in the medium.

### References

1. Chamberlain CG, McAvoy JW. Induction of lens fibre differentiation by acidic and basic fibroblast growth factor (FGF). *Growth Factors.* 1989;1:125-134.
2. McAvoy JW, Chamberlain CG. Fibroblast growth factor (FGF) induces different responses in lens epithelial cells depending on its concentration. *Development.* 1989;107:221-228.
3. McAvoy JW, Chamberlain CG, de Iongh RU, Richardson NA, Lovicu FJ. The role of fibroblast growth factor in eye lens development. *Ann NY Acad Sci.* 1991;638:256-274.
4. Hales A, Chamberlain CG, McAvoy JW. Measurement of lens epithelial cell migration on a laminin substratum using image analysis. *J Comput Assist Microscopy.* 1992;4:135-139.
5. McAvoy JW, Fernon VTP. Neural retinas promote cell division and fibre differentiation in lens epithelial explants. *Curr Eye Res.* 1984;3:827-834.
6. Parmigiani CM, McAvoy JW. The roles of laminin and fibronectin in the development of the lens capsule. Curr *Eye Res.* 1991;10:501-511.
7. Lovicu FJ, McAvoy JW. The age of rats affects the response of lens epithelial explants to fibroblast growth factor - an ultrastructural analysis. *Invest Ophthalmol Visual Sci.* 1992;33:2269-2278.
8. McAvoy JW. Cell lineage analysis of lens epithelial cells induced to differentiate into fibres. *Exp Eye Res.* 1988;47:869-883.
9. Lovicu FJ , McAvoy JW. Structural analysis of lens epithelial explants induced to differentiate into fibres by fibroblast growth factor (FGF) . *Exp Eye Res.* 1989;49:479-494.
10. Wyllie AH, Kerr JFR, Currie AR. Cell death: the significance of apoptosis. *Internat Rev Cytol.* 1980;68:251-299.
11. Williams GT, Smith AS, McCarthy NJ, Grimes EA. Apoptosis: final control point in cell biology. *Trends Cell Biol.* 1992;2:263-267.
12. Mohan PS, Spiro RG. Macromolecular organization of basement membranes. Characterization and comparison of glomerular basement membrane and lens capsule components by immunochemical and lectin affinity procedures. *J Biol* Chem. 1986;261:4328-4336.
13. Novotny GEK, Pau H. Myofibroblast-like cells in human anterior capsular cataract. *Virchows Arch [Pathol Anatl]*. 1984;404:393-401.
14. Eshagian J. Human posterior subcapsular cataracts. *Trans Ophthal Soc UK.* 1982; 102:364-368.
15. Eshagian J, Streeten B. Human posterior subcapsular cataract - an ultrastructural study of the posteriorly migrating cells. *Arch Ophthalmol.* 1980;98:134-143.
16. Green WR, McDonnell PJ. Opacification of the posterior capsule. *Trans Ophthalmol Soc UK*. 1985;104:727-739.
17. Schulz MW, Chamberlain CG, de Iongh RU, McAvoy JW. Acidic and basic FGF in ocular media and lens: implications for lens polarity and growth patterns. Development 1993;118:117-126.
18. Yamaguchi Y, Mann DM, Ruoslahti E. Negative regulation of TGFβ by the proteoglycan decorin. *Nature*. 1990;346:281-284.
19. McCaffrey TA, Falcone DJ, Du B. TGFβ1 is a heparan-binding protein: identification of putative heparan-binding regions and isolation of heparans with varying affinities for TGFβ1. *J. Cell. Physiol.* 1992:152;430-440.
20. Hales AM, Schulz MW, Chamberlain CG, McAvoy JM. TGF-β1 induces lens cells to accumulate α-smooth muscle actin, a marker for subcapsular cataracts. Curr *Eye Res.,* in press.
21. Twining SS, Fukuchi T, Yue BYJT, Wilson PM, Zhou X, Loushin G. α₂-macroglobulin is present in and synthesised by the cornea. *Invest. Ophthalmol. Vis. Sci .* 1994:35;3226-3233.
22. Beebe DC, Latker CH, Jebens HAH, Johnson MC, Feagans DE, Feinberg RN. Transport and steady-state concentrations of plasma proteins in the vitreous humor of the chicken embryo: Implications for the mechanism of eye growth during early development. *Dev Biol.* 1986;114:361-368.
23. Ando H, Twining SS, Yue BYJT, Zhou X, Fini ME, Kaiya T, Higginbottom EJ, Sugar J. MMPS and proteinase inhibitors in the human aqueous humour. *Invest. Ophthalmol. Vis. Sci.* 1993:34;3541-3548.
24. Hagedorn M, Esser P, Wiedeman P, Heimann K. Tenascin and decorin in epiretinal membranes of proliferative vitreoretinopathy and proliferative diabetic retinopathy. *German J Ophthalmol.* 1993;2:28-31.
25. Kamei A, Totani A. Isolation and characterisation of minor glycosaminoglycans in the rabbit vitreous. *Biochem Biophys Res Comm.* 1982;109:881-887.
26. Paralkar VM, Vukicevic S, Reddi AH. TGFβ binds collagen type IV of basement membrane matrix: implications for development. *Dev. Biol.* 1991:143,303-308.
27. LaMarre J, Wollenberg GK, Gonias SL, Hayes MA. Cytokine binding and clearance properties of proteinase-activated α₂-macroglobulins. *Lab Invest.* 1991;65:3-14.
28. Choi HU, Johnson TL, Pal S, Tang L-H, Rosenberg L and Neame PJ. Characterisation of the dermatan sulfate proteoglycans, DS-PGI and DS-PGII, from bovine articular cartilage and skin isolated by octyl-Sepharose chromatography. *J. Biol. Chem.* 1989; 264:2876-2884.
29. Yanagishita M, Midura RJ and Hascall VC. Proteoglycans: Isolation and purification from tissue culture. *Methods in Enzymology* 1987 138:279-289.

## Claims

1. The use of one or more inhibitors of TGFβ in the manufacture of a pharmaceutical formulation for preventing or controlling cataract or aftercataract formation.

2. Use according to claim 1 wherein the inhibitors of TGFβ are selected from proteins, glycoproteins and proteoglycans.

3. Use according to claim 2 wherein the protein inhibitors of TGFβ are selected from antibodies and peptide growth factors.

4. Use according to claim 2 wherein the glycoprotein inhibitors of TGFβ are selected from α₂-macroglobulin, laminin and collagen.

5. Use according to claim 2 wherein the proteoglycan inhibitors of TGFβ are selected from decorin, heparan sulfate proteoglycans and biglycan.

6. A topical ophthalmological formulation for topical application to the surface of the eye for preventing or controlling cataract or aftercataract formation in the eye, said formulation comprising one or more inhibitors of TGFβ in an ophthalmologically acceptable carrier.

7. An ophthalmological irrigation solution or viscoelastic solution for preventing or controlling cataract or aftercataract formation in the eye comprising one or more inhibitors of TGFβ in an irrigation solution or viscoelastic solution.

8. A formulation according to claim 6 or 7 wherein the inhibitors of TGFβ are as defined in claim 2, 3, 4 or 5.

9. Use of one or more inhibitors of TGFβ in the manufacture of a synthetic lens for implantation into the lens capsule of the eye following the removal of lens cells from the lens capsule.

10. Use according to claim 9 wherein the TGFβ inhibitors are as defined in claim 2, 3, 4 or 5.

11. A synthetic lens implant for implantation into the lens capsule of the eye following removal of lens cells from the lens capsule, said lens implant being coated with one or more TGFβ inhibitors.

12. A lens implant according to claim 11, wherein the TGFβ inhibitors are as defined in claim 2, 3, 4 or 5.

## Patentansprüche

1. Die Anwendung von einem oder mehreren Inhibitor(en) von TGFβ in Verbindung mit der Herstellung einer pharmazeutischen Formulierung zur Verhinderung oder Kontrolle von Katarakt- oder Sekundärkataraktbildung.

2. Anwendung gemäß Anspruch 1, worin die Inhibitore von TGFβ ausgewählt sind aus Proteinen, Glycoproteinen und Proteoglycanen.

3. Anwendung gemäß Anspruch 2, worin die Proteininhibitore von TGFβ ausgewählt sind aus Antikörpern und Peptidwachstumfaktoren.

4. Anwendung gemäß Anspruch 2, worin die Glycoproteininhibitore von TGFβ ausgewählt sind aus α₂-Macroglobulin, Laminin und Kollagen.

5. Anwendung gemäß Anspruch 2, worin die Proteoglycanininhibitore von TGFβ ausgewählt sind aus Decorin, Heparansulfatproteoglycanen und Biglycan.

6. Eine topische ophthalmologische Formulierung für topische Anwendung an der Augenoberfläche zur Verhinderung oder Kontrolle von Katarakt- oder Sekundärkataraktbildung Im Auge, welche Formulierung einen oder mehrere Inhibitor(e) von TGFβ in einem ophthalmologisch akzeptablen Träger umfasst.

7. Eine ophthalmologische Spülungslösung oder viskoelastische Lösung zur Verhinderung oder Kontrolle von Katarakt- oder Sekundärkataraktbildung im Auge umfassend einen oder mehrere Inhibitor(e) von TGFβ in einer Spülungslösung oder viskoelastischen Lösung.

8. Eine Formulierung gemäß Anspruch 6 oder 7, worin die Inhibitore von TGFβ so sind wie im Anspruch 2, 3, 4 oder 5 definiert.

9. Anwendung von einem oder mehreren Inhibitor(en) von TGFβ zur Herstellung einer synthetischen Linse für die Implantation in die Linsenkapsel des Auges nach Entfernen von Linsenzellen von der Linsenkapsel.

10. Anwendung gemaß Anspruch 9, worin die TGFβ-Inhibitore so sind wie im Anspruch 2, 3, 4 oder 5 definiert.

11. Ein synthetisches Linsenimplantat für die Implantation in die Linsenkapsel des Auges nach Entfernen der Linsenzellen von der Linsenkapsel, welches Linsenimplantat mit einem oder mehreren TGFβ-Inhibitor(en) belegt ist.

12. Ein Linsenimplantat gemäß Anspruch 11, worin die TGFβ-Inhibitore so sind wie im Anspruch 2, 3, 4 oder 5 definiert.

## Revendications

1. L'usage d'un ou plusieurs inhibiteurs de TGFβ dans la manufacture d'une formulation pharmaceutique pour prévenir ou contrôler la formation de cataracte ou d'après cataracte.

2. Usage selon la revendication 1 dans lequel les inhibiteurs de TGFβ sont choisis parmi les protéines, les glycoprotéines et les protéoglycanes.

3. Usage selon la revendication 2 dans lequel les protéines inhibitrices de TGFβ sont choisis parmi les anticorps et les facteurs de croissance peptidiques.

4. Usage selon la revendication 2 dans lequel les glycoprotéines inhibitrices de TGFβ sont choisis parmi l'α₂-macroglobuline, la laminine et le collagène.

5. Usage selon la revendication 2 dans lequel les protéoglycanes inhibiteurs de TGFβ sont choisis parmi la décorine, les protéoglycanes de sulfate d'héparane et les biglycanes.

6. Formulation ophthalmologique d'actualité pour application d'actualité à la surface de l'oeil pour prévenir ou contrôler la formation de cataracte ou d'après cataracte dans l'oeil, ladite formulation comprenant un ou plusieurs inhibiteurs de TGFβ dans un porteur ophthalmologiquement acceptable.

7. Solution ophthalmologique d'irrigation ou solution viscoélastique pour prévenir ou contrôler la formation de cataracte ou d'après cataracte dans l'oeil comprenant un ou plusieurs inhibiteurs de TGFβ dans une solution d'irrigation ou une solution viscoélastique.

8. Formulation selon les revendications 6 ou 7 dans laquelle les inhibiteurs de TGFβ sont tels que définis dans les revendications 2, 3, 4 ou 5.

9. Usage d'un ou plusieurs inhibiteurs de TGFβ dans la manufacture d'un cristallin synthétique pour implantation dans la capsule du cristallin de l'oeil suivant l'ablation de cellules du cristallin de la capsule du cristallin.

10. Usage selon la revendication 9 dans lequel les inhibiteurs de TGFβ sont tels que définis dans les revendications 2, 3, 4 ou 5.

11. Cristallin synthétique pour implantation dans la capsule du cristallin de l'oeil suivant l'ablation de cellules du cristallin de la capsule du cristallin, ledit implant de cristallin étant enrobé d'un ou plusieurs inhibiteurs de TGFβ.

12. Un implant de cristallin selon la revendication 11, dans lequel les inhibiteurs de TGFβ sont tels que définis dans les revendications 2, 3, 4 ou 5.
